# EUROPEAN PATENT APPLICATION

(11) **EP 0 879 594 A1**
(43) Date of publication of application: **25.11.1998**
(21) Application number: 97870132.4
(22) Date of filing: 09.09.1997
(51) Int. Cl.: A61K 7/50, A61K 7/48

(54) **Soap composition**

(30) Priority: 20.05.1997 BE 9700439
(71) Applicant: "Galenco", 3583 Paal (BE)
(72) Inventor: Vandamme, Patricia, 1702 Groot-Bijgaarden (BE); Caers, Els, 3945 Ham (BE)
(74) Representative: Claeys, Pierre

(57) **Abstract**

The present invention relates to a soap composition comprising a surface active compound and an oil, whereby said oil is present in an amount of at least 30 percent by weight with respect to the total weight of the composition, and said oil is a mixture of (a) 0-100 percent by weight with respect to the total weight of the oil, of an ester which is the condensation product of an alcohol with one or more fatty acids comprising 8-20 C-atoms, preferably 8-10 C-atoms, and (b) 100-0 percent by weight with respect to the total weight of the oil, of a hydrocarbon oil. The ester is preferably pentaerytrityl-tetra-caprylate/caprate. The hydrocarbon oil is preferably a paraffin oil or a poly alfaolefin, preferably polydecene.

## Description

The present invention relates to a soap composition comprising a surface active component and an oil.

Such compositions are used for a simultaneous cleaning and hydration of the skin during showering.

Upon a cleaning of the skin with water and a surface active compound, such as for example soap, the impurities present on the skin are removed with the water. However, simultaneously with the removal of the impurities, the balance within the protective hydrolipid film of the skin, is upset. In case of a healthy skin, this balance will be repaired without any problem. In case of a skin with a somewhat distorted metabolism, this repair mechanism does not function optimally. The skin is only capable of a limited regeneration. This can involve an exceeding dehydration and the formation of a rough, dry skin.

Such a soap composition is known from EP-A-691.127. The soap composition disclosed in EP-A-691.127 is essentially free of water and contains at least 45 percent by weight of one or more vegetable oil components with a high triglyceride content, not more than 55 percent by weight of surface active components, anti oxidising agents and, if so desired, further cosmetic additives.

The soap composition disclosed in EP-A-691.127 however has the disadvantage that it brings about an unpleasant dry feeling of the skin.

It is the aim of the present invention to provide a soap composition which is capable of avoiding this unpleasant dry feeling.

This is achieved according to the invention in that the composition comprises at least 30 percent by weight, with respect to the total weight of the composition of said oil, and said oil is a mixture of (a) 0-100 percent by weight with respect to the total weight of the oil, of an ester which is the condensation product of an alcohol with one or more fatty acids comprising 8-20 C-atoms, preferably 8-10 C-atoms, and (b) 100-0 percent by weight with respect to the total weight of the oil, of a hydrocarbon oil.

Surprisingly it has found that the soap composition of the present invention, can substantially completely be resorbed by the skin. In that way, the unpleasant fatty feeling of the skin can be avoided. This substantially complete resorption by the skin offers the possibility to obtain an improved hydration of the skin, without thereby involving an unpleasant fatty feeling of the skin. Such a soap composition allows to coat the skin, from the inside with an oil, and to prevent a dehydration for example by the evaporation of water, from the inside of the skin.

The soap composition of the invention moreover shows an improved spreading ability than the known soap composition. In that way the oil component can be spread out uniformly over the entire surface of the skin, so that a uniform hydration of the skin can be obtained.

Because the oil component of the soap composition of the present invention comprises mainly synthetic oils, it is also possible to control the composition of the oil component and to obtain a soap composition with a constant composition and consequently a constant quality and constant hydrating ability. With the known composition on the other hand, the composition and quality can vary significantly, because the oil components are natural vegetable oils. The composition, and more particularly, the triglyceride content of such natural oils can vary significantly with the origin, climate and supplier of the oil.

The synthetic oils used in the composition of this invention moreover show an improved resistance to oxidation upon exposure to air, and show an improved stability with respect to the vegetable oils used in the known composition. As a consequence, according to the present invention, the use of anti-oxidising agents can be significantly reduced. Vegetable oils mostly show a fast oxidation upon exposure to air, following which they become rancid and start spreading a nasty smell. To prevent these oils from turning rancid, the known composition comprises a sufficient amount of anti-oxidising agents. The soap composition of this invention on the other hand has a simple, balanced formulae, and presents an improved stability. Due to the use of synthetic oils, it is further possible to obtain a soap composition with a constant colour.

In a first preferred embodiment of the invention, the oil comprises (a) 45-75 percent by weight, preferably 55-65 percent by weight of said ester and (b) 30-10 percent by weight, preferably 25-15 percent by weight of a hydrocarbon oil.

The ester is preferably the condensation product of a fatty acid with an alcohol, whereby the alcohol is selected from the group of polyhydric alcohols and adducts of an alkoxide with a fatty acid alcohol.

According to a more preferred embodiment, the polyhydric alcohol is selected from the group of polyhydric alcohols with 1-10 C atoms and 2-6 OH groups. The ester is most preferably pentaerythritol.

The adduct is preferably an adduct of an alcoxide and a fatty alcohol, which alcohol corresponds to the formula: 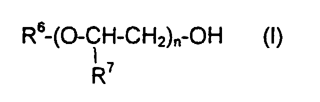
- wherein: R⁶ is a fatty acid group with 12-20 C atoms
R⁷ is H, or an alkylgroup with 1-5 C atoms.

The ester is most preferably pentaerytrityl-tetra-capryl/capraat, which corresponds to the formula: 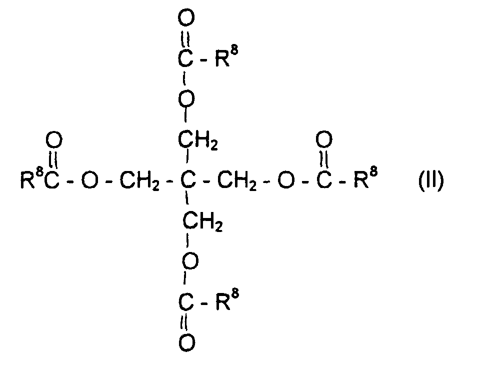
- wherein: R⁸ is a mixture of a capryl and caprine acid residu.

Petaerytritol esters have the advantage that they almost don't involve any allergic reactions or irritations of the skin.

The hydrocarbon oil used according to the invention is preferably a paraffin oil, more preferred a poly alpha olefin. The poly alpha olefin is preferably polydecene, which corresponds to the chemical formula given below:

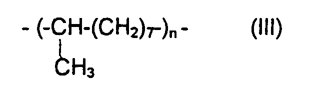

The polydecene oil is an oil which is frequently added to food, and which can be obtained in high purities. Polydecene oil is a non toxic oil and does almost not involve any irritation of the skin.

The soap composition according to this invention preferably also contains an oil with a high linolic and/or linoleic acid content, more preferably, oenothera biennis. In a healthy skin, linolic acid is transformed to γ-linoleic acid, by enzymes (delta 6 desaturase) present in the skin. γ-linoleic acid is capable of improving the penetration of the soap composition in to the skin and plays an essential role in the binding of the water to the stratum comeum. γ-linoleic acid is incorporated into the metabolic active cells in the epidermis, in such a way that a water binding double layer is formed in the inter cellular space of the stratum comeum, which can lead to a skin with an improved hydration. By supplying additional γ-linoleic acid to the skin, it becomes possible to prevent or slow down the formation of a dry rough skin with grooves, and to slow down or prevent inflammations of the skin.

The soap composition of the present invention can further contain the usual surface active compounds for cleaning the skin. Preferably, the surface active compounds comprise one or more components of the group of fatty alcohol ethoxylates, fatty alcohol sulphates, amides of the fatty alcohol sulphates, fatty alcohol ether sulphates, amides of the fatty alcohol ether sulphates, fatty acids of mono ethyl- and/or di-ethanol amide. The fatty alcohol sulphates and fatty alcohol ether sulphates preferably correspond tot the formula given below: 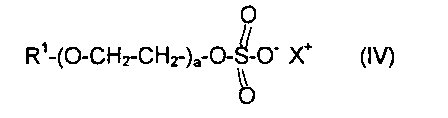
- wherein: a is 0-10, preferably 1 to 5
R¹ is a branched or straight alkyl group with 6-24 carbon atoms
X⁺ is a alkali metal ion or an ammonium ion which can contain one or more alkyl- and/or hydroxy alkyl substituents

The amides used according to the present invention preferably correspond tot the formula given below: 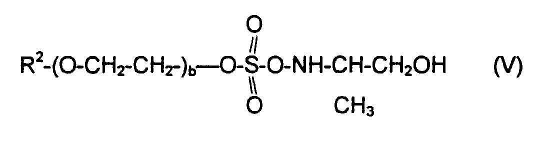
- wherein: b is 0-10, preferably 1-5,
R² is a straight or branched alkyl group with 6-24 carbon atoms.

Most preferably, the composition according to the invention contains MIPA laureth sulphate.

The fatty alcohol ethoxylates used according to the present invention preferably correspond to the formula

R³ - (O-CH₂-CH₂)_{c}-OH (VI)

- wherein: c is 1-45, preferably 1-10,
R³ is a straight or a branched alkyl group with 6-24 C atoms.

The surface active compounds according to the invention preferably contain laureth-4.

The fatty mono and di-ethanol amides used according to the invention, respectively preferably correspond to the structural formulas given below: 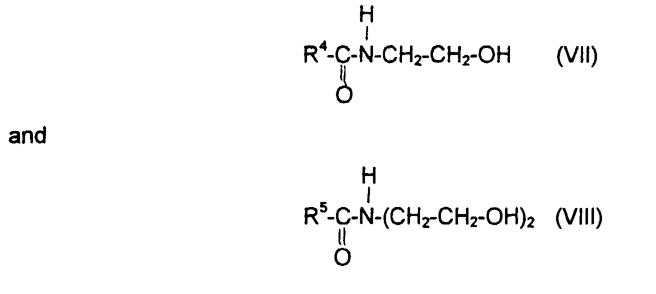
- wherein: R⁴ and R⁵ are branched or non branched alkyl- or alkenyl groups, with 6-24 carbon atoms.

The preferred di-ethanol amide according to the invention is the coconut fatty acid di-ethanol amide (coco amide DEA). The most important components of natural coco amide are 44-51 wt.% of lauric acid, 13-18 wt.% of myristic acid, 8-10 wt.% of palmic acid, 6-9 wt.% of caprylic acid, 6-10 wt.% of caprin acid, 5-8 wt.% of oil acid, 1-3 wt.% of stearic acid, 0-2 wt. % of linolic acid and 0-1 wt.% of capronic acid.

According to the invention, preferably a mixture of MIPA laureth sulphate, laureth-4, and coco amid DEA are used. Such mixtures are for example sold by Zschimmer & Schwarz, Lahnstein Rhein, Germany as ZETESOL 100, or by Henkel Kga, Düsseldorf, Germany as TEXAPON WW99. These surface active compounds provide a soap composition giving a creamy foam.

The soap composition according to the invention can further comprise the usual perfumes, cosmetic additives, additives capable of preventing inflammations and improving the healing of wounds.

The soap composition according to the invention is substantially free of water. The individual components of the soap composition of the present invention are also substantially free of water.

The soap composition according to the invention presents simultaneous good hydrating and skin regulating properties and shows a positive influence to the general condition of the skin, without thereby negatively influencing the cleaning properties of the soap composition. By using the soap composition of the present invention, a smooth, soft and souple feeling skin can be obtained, whereby the unpleasant dry feeling of the skin can be prevented.

The invention is further elucidated by the following example.

### Example.

A soap composition for use as shower composition was prepared, which contains the components summarised below in the indicated amounts:

| Component | Amount (wt. %) |
|---|---|
| Pentaerytrityl Tetra Caprylate/Caprate | 35,00 |
| Polydecene | 20,00 |
| MIPA laurethsulphate | 15,00 |
| Laureth-4 | 15,00 |
| Cocoamide DEA | 7,00 |
| Oenothera Biennis | 5,00 |

The composition can further contain anti-oxidising agents (approximately 0.25 wt.%). The amount of anti-oxidising agents in the composition can significantly be decreased by the synergetic activity of the anti-oxidising agents used, namely ascorbyl palmitate, rosemarinus officials and tocopheryl acetate. The composition further comprises perfume, and the usual additives, with for example inflammation preventing and wound healing capacities.

## Claims

1. Soap composition comprising a surface active compound and an oil, characterised in that said composition comprises at least 30 percent by weight of an oil, with respect to the total weight of the composition, and said oil is a mixture of (a) 0-100 percent by weight with respect to the total weight of the oil, of an ester which is the condensation product of an alcohol with one or more fatty acids comprising 8-20 C-atoms, preferably 8-10 C-atoms, and (b) 100-0 percent by weight with respect to the total weight of the oil, of a hydrocarbon oil.

2. Soap composition as claimed in claim 1, characterised in that said oil comprises (a) 45-75 percent by weight with respect to the total weight of the oil, preferably 55-65 percent by weight, of said ester, and (b) 30-10 percent by weight with respect to the total weight of the oil preferably 25-15 percent by weight, of said hydrocarbon oil.

3. Soap composition as claimed in any one of claims 1 or 2, characterised in that said alcohol is chosen from the group of polyhydric alcohols and adducts of an alkoxide with a fatty alcohol.

4. Soap composition as claimed in claim 3, characterised in that said polyhydric alcohol is an alcohol with 1-10 C-atoms and 2-6 OH groups, preferably pentaerythritol.

5. Soap composition as claimed in claim 3, characterised in that said adduct is an adduct of an alkoxide and a fatty alcohol, corresponding to the formula (I): 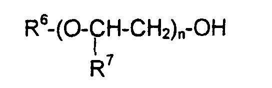
wherein R⁶ is a fatty acid group with 12-20 C atoms
R⁷ is H, or an alkylgroup with 1-5 C atoms

6. Soap composition as claimed in any one of claims 1 to 4, characterised in that said ester is a pentaerytrityl-tetra-capryl/capraat, which corresponds to the formula (II): 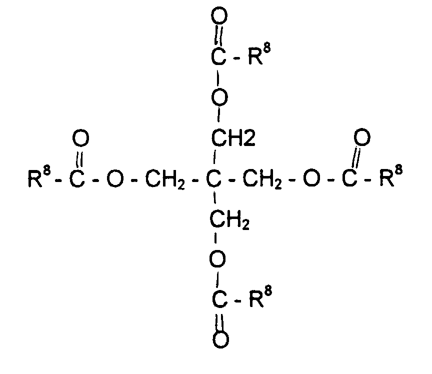
wherein R⁸ is a mixture of a capryl and caprine acid residu.

7. Soap composition as claimed in any one of claims 1 to 6, characterised in that said hydrocarbon oil is a paraffin oil or a poly alfaolefin, preferably polydecene.

8. Soap composition as claimed in any one of claims 1 to 7, characterised in that said composition comprises at least 3 percent by weight of a linoleic rich oil, preferably oenothera biennis.

9. Soap composition as claimed in any one of claims 1 to 8, characterised in that said composition comprises not more than 70 percent by weight with respect to the total weight of the composition, of a surface active compound, which surface active compound comprises one or more components from the group of fatty alcohol ethoxylates, fatty alcohol sulphates, amides of the fatty alcohol sulphates, fatty alcohol ether sulphates, amides of the fatty alcohol ether sulphates, fatty acids of mono ehtyl- and/or diethanol amide.

10. Soap composition as claimed in claim 9, characterised in that said surface active compound is a mixture of MIPA laureth sulphate, laureth-4 and coconut fatty acid diamide.
